# EUROPEAN PATENT APPLICATION

(11) **EP 3 868 282 A1**
(43) Date of publication of application: **25.08.2021**
(21) Application number: 19874529.1
(22) Date of filing: 07.10.2019
(51) Int. Cl.: A61B 5/00, G02B 21/00, G02B 21/06, G02B 21/24

(54) **MICROSCOPIC IMAGE ACQUISITION SYSTEM OF IN-VIVO DEEP TISSUE AND MICROSCOPIC IMAGE PROVIDING METHOD THEREFOR**

(30) Priority: 17.10.2018 KR 20180123869; 05.12.2018 KR 20180155498
(71) Applicant: Korea Advanced Institute of Science and Technology, Daejeon 34141 (KR)
(72) Inventor: KIM, Pilhan, Daejeon 34141 (KR); KONG, Eunji, Daejeon 34141 (KR); AHN, Jin Hyo, Daejeon 34141 (KR)
(74) Representative: Verriest, Philippe
(86) International application number: PCT/KR2019/013128
(87) International publication number: WO 2020/080721

(57) **Abstract**

A microscopic image acquisition system includes: a microscope apparatus that scans light of multiple wavelengths, transmits the light to an objective lens, and detects a fluorescence signal reflected at a living tissue and transmitted to the objective lens; an endoscope probe including lenses, which is inserted into an implantable device implanted in a living body, transmits an incident light from the objective lens to a side opening at a lower end, and transmits a fluorescence signal emitted from the living tissue labeled with a fluorescent material to the objective lens; and a rotatable probe moving device that fixes the endoscope probe to rotate or vertically move the fixed endoscope probe.

## Description

### [Technical Field]

The present invention relates to a bio-imaging technology.

### [Background Art]

An endomicroscopy using a fluorescence signal was developed to observe phenomena at the cellular and molecular level occurring in the deep living tissues. However, the tissues of the brain or the small intestine are simultaneously organically connected while performing special functions of each microstructure, and are distributed over a wider and deeper area compared to other tissues. As a result, there is a limit in imaging the brain tissues and others with the conventional optical imaging technology. In order to overcome this limitation, researches on fluorescence endoscopy to image a front view or a side view of deep tissues by inserting an optical probe into a living body have been performed.

However, there is a problem in that inserting repeatedly the optical probe at the same point causes damage to the tissue. Therefore, it is difficult to stereoscopically and repeatedly image the deep tissues of a living body at the cellular level with the conventional fluorescence endoscopy.

In spite of such limitations, in order to accurately observe phenomena occurring in deep living tissues, it is necessary to acquire a microscopic image with maintaining the physiological and behavioral characteristics of a living animal. Additionally, it is necessary to repeatedly perform cell-level high-resolution imaging of the deep tissues in order to observe the changes in tissues over time. Thus, a new technology for researching in vivo microscopic imaging is required.

### [Disclosure]

### [Technical Problem]

The present invention relates to a system for in vivo microscopic imaging of deep tissue, and a microscopic imaging method.

An aspect is to provide a microscopic imaging system that stereoscopically and repeatedly acquires microscopic images of a deep living tissue. In particular, a wall glass capillary is implanted in a living animal and an endoscope probe which is inserted into the wall glass capillary and then vertically moves and rotates is utilized.

Another aspect is to provide a microscopic image acquisition system in which an endoscope probe connected with a microscope apparatus emits a light of multiple wavelengths while vertically moving and rotating, when the endoscope probe is implanted in a wall glass capillary implanted in a living animal, and the microscope apparatus obtains a fluorescence signal emitted from a deep tissue to generate a microscopic image.

Yet another aspect is to implant a square-type window chamber in a living animal and to use an endoscope probe which is inserted into the square-type window chamber and then moves vertically and rotates.

### [Technical Solution]

According to an embodiment, a microscopic image acquisition system is provided. The microscopic image acquisition system includes: a microscope apparatus that scans light of multiple wavelengths, transmits the light to an objective lens, and detects a fluorescence signal reflected at a living tissue and transmitted to the objective lens; an endoscope probe including lenses, which is inserted into an implantable device implanted in a living body, transmits an incident light from the objective lens to a side opening at a lower end, and transmits a fluorescence signal emitted from the living tissue labeled with a fluorescent material to the objective lens, and a rotatable probe moving device that fixes the endoscope probe to rotate or vertically move the fixed endoscope probe.

The microscopic image acquisition system may include a gradient index lens (GRIN lens) to transmit a light having passed through the objective lens, and a microprism to emit laterally a light transmitted from the GRIN lens.

The endoscope probe may include a coupling lens, a relay lens, and an imaging lens.

The rotatable probe moving device may include a probe holder to fix the endoscope probe, a rotation mount to interlock with the endoscope probe and to rotate the endoscope probe, and a translation stage to vertically and horizontally move the rotation frame.

The rotatable probe moving device may rotate the endoscope probe fixed to the probe holder by moving the rotation frame and may vertically move the endoscope probe by moving the translation stage.

The microscopic image acquisition system may further include a stage implemented with a stereotactic system for an animal having implanted the implantable device, and a plate to which the implantable device is attached may be fixed to the stage.

The implantable device may be implanted in the animal with being fixed to the plate.

The implantable device may be filled with a liquid that has a specific refractive index to control refraction of a light emitted by the endoscope probe.

The microscope apparatus may include laser light sources to emit the light of the multiple wavelengths, a scanner to scan lights from the laser light sources using a plurality of mirrors, the objective lens that transmits a light having passed through the scanner to the endoscope probe, photodetectors that detect fluorescence signals having passed through pinholes formed on a confocal plane by wavelength, among the fluorescence signals transmitted from the endoscope probe, and a computing device to generate an image by combining signals output from the photodetectors.

The microscope apparatus may include a multi-photon microscopy.

The computing device may generate a fluorescence image, expressing the fluorescent material labeled on a living tissue of a specific depth and excited by the laser light sources while the endoscope probe rotates.

The computing device may stereoscopically generate a fluorescence image of the living tissue based on a rotational and a vertical movement position of the endoscope probe.

The implantable device may be a wall glass capillary or a square-type window chamber that are implemented with a passage into which the endoscope probe is inserted.

According to an embodiment, a method for acquiring a microscopic image by a microscopic image acquisition system is provided. The method includes emitting a light of multiple wavelengths generated by a microscope apparatus into a living tissue through an endoscope probe; and generating a fluorescence image using a fluorescence signal emitted from the living tissue, wherein the fluorescence image expresses a fluorescent material labeled on the living tissue and excited by a multi-wavelength laser. The light of multiple wavelengths is transmitted from an objective lens of the microscope apparatus to a lens of the endoscope probe and emitted to a living tissue around a side opening of the endoscope probe, and the fluorescence signal is transmitted from the lens of the endoscope probe to the objective lens. The endoscope probe is inserted into a wall glass capillary or a square-type window chamber implanted in the living tissue, and rotates and moves vertically.

Generating the fluorescence image may include stereoscopically generating a fluorescence image of the living tissue based on rotational and vertical movement positions of the endoscope probe.

The microscope apparatus may include a confocal microscopy or a multi-photon microscopy.

### [Advantageous Effects]

According to an embodiment, since fluorescence imaging is performed on the living animal tissue, the phenomena occurring in the living tissue maintaining physiological and behavioral characteristics can be observed accurately and in real time.

According to an embodiment, since it is possible to repeatedly acquire microscopic images of the same deep tissue in a living body, changes in the tissue of the living body over time can be observed repeatedly and in the long term.

According to an embodiment, since the endoscope probe vertically moves and rotates, an extended image of a large area can be obtained, and a three-dimensional image of not only two dimensions but also three dimensions can be obtained.

According to an embodiment, it is possible to stably and repeatedly access deep tissues inaccessible by conventional fluorescence endoscopes over a long period of time. Accordingly, according to an embodiment, various diseases or biological phenomena occurring in various deep tissues including brain tissues can be more accurately identified over the long term.

### [Description of the Drawings]

FIG. 1 is a configuration diagram of a microscopic image acquisition system according to an embodiment.
FIG. 2 is an exemplary endoscope probe according to an embodiment.
FIG. 3 is an exemplary rotatable probe moving device according to an embodiment.
FIG. 4 is an exemplary confocal microscope device according to an embodiment.
Fig. 5 is a drawing illustrating a skull-attached plate for implantation of a glass tube in a living body according to an embodiment.
FIG. 6 is a diagram illustrating a case where a mouse is fixed to a stage of a microscopic image acquisition system according to an embodiment.
FIG. 7 is an exemplary image of a mouse brain tissue acquired by a microscopic image acquisition system according to an embodiment.
FIG. 8 is an exemplary large-area fluorescence image acquired according to an embodiment.
FIG. 9 is a flowchart of a microscopic image acquisition method according to an embodiment.
FIG. 10 is an exemplary implantable device implanted in a living body according to another embodiment.

### [Mode for Invention]

Hereinafter, embodiments of the present invention will be described in detail with reference to the attached drawings so that the person of ordinary skill in the art may easily implement the present invention. However, the present invention may be implemented in various different forms and is not limited to the examples described herein. In the drawings, elements irrelevant to the description of the present invention are omitted for simplicity of explanation, and like reference numerals designate like elements throughout the specification.

Throughout the specification, when a part is referred to "include" a certain element, it means that it may further include other elements rather than exclude other elements, unless specifically indicates otherwise. In addition, the terms such as "... unit", "... group", "... module", and the like in the description refer to units that process at least one function or operation, which may be implemented with a hardware, a software or a combination thereof.

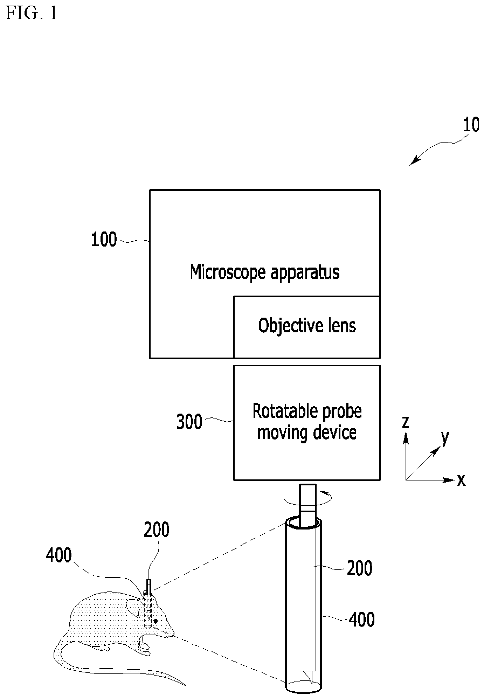

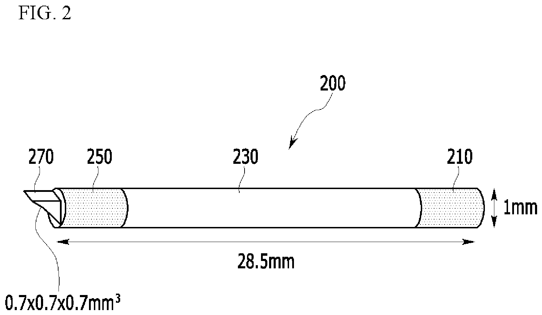

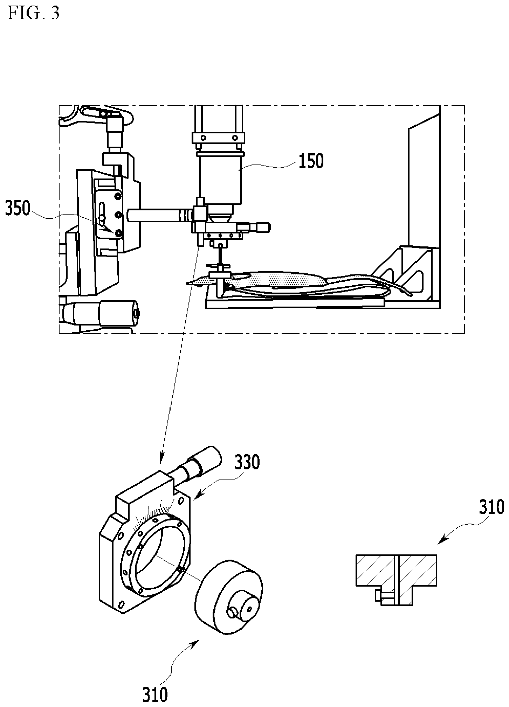

FIG. 1 is a configuration diagram of a microscopic image acquisition system according to an embodiment, FIG. 2 is an exemplary endoscope probe according to an embodiment, and FIG. 3 is an exemplary rotatable probe moving device according to an embodiment.

Referring to FIG. 1, a microscopic image acquisition system 10 includes a microscope apparatus 100, an endoscope probe 200 of a certain length, and a rotatable probe moving device 300. The rotatable probe moving device 300 fixes the endoscope probe 200 so that incident light from an objective lens of the microscope apparatus 100 is transmitted to the endoscope probe 200 and the endoscope probe 200 is operated to move vertically or to rotate.

A wall glass capillary 400 is implanted in a tissue of a living animal. The animal with the wall glass capillary 400 implanted is fixed on a motorized stage implemented with a stereotactic system. Thereafter, the endoscope probe 200 fixed to the rotatable probe moving device 300 is inserted into the wall glass capillary 400 implanted in the tissue of the living animal. The endoscope probe 200 inserted into the wall glass capillary 400 emits light into a deep tissue while being vertically moved and rotated by the rotatable probe moving device 300, and transmits light (fluorescence signal) emitted from the deep tissue labeled (marked) with a fluorescent material to the microscope apparatus 100.

A position relationship between the objective lens of the microscope apparatus 100 and the endoscope probe 200, and a position relationship between the endoscope probe 200 and the wall glass capillary 400 are designed to enable fine adjustment.

The microscope apparatus 100 may be implemented with a microscope of various types and, for example, may use a confocal microscopy or a multi-photon microscopy. The microscope apparatus 100 makes the fluorescent material emit light with a laser scanning method and detects light transmitted from the endoscope probe 200 to generate a two-dimensional (2D) or three-dimensional (3D) image.

The endoscope probe 200 is a side-view endoscope that emits light in the direction of a wall of the wall glass capillary 400. The endoscope probe 200 transmits the light having passed through the objective lens of the microscope apparatus 100 to an imaging area inside the living tissue, and transmits backwards the light reflected from inside of the living tissue to the objective lens. The shape of the endoscope probe 200 may be various. However, in the present invention, the endoscope probe 200 may have the shape of a rod or a needle that can be inserted into the wall glass capillary 400 and can accommodate a gradient index lens (GRIN lens).

Since the endoscope probe 200 is inserted into the wall glass capillary 400 implanted in the animal tissue, the microscopic image acquisition system 10 can repeatedly generate microscopic images of a deep living tissue without damaging the tissue.

The upper end of the endoscope probe 200 is combined with the rotatable probe moving device 300. Further, the endoscope probe 200 includes a lens structure that transmits light incident from the objective lens to the side opening at the lower end.

Referring to FIG. 2, the endoscope probe 200 may be manufactured with a GRIN lens having a micro-prism attached to the lower end thereof for side imaging. Optical conditions of the GRIN lens are optimized in consideration of conditions suitable for observing biological microstructures, including minimal invasive. The optical conditions include a diameter of the GRIN lens (e.g., 1mm), a length (e.g., 28.5mm), an operation distance, a size of the attached micro-prism (e.g., 0.7x0.7x0.7 mm³), and the like. The inside of the endoscope probe 200 may include a triplet lens structure composed of a coupling lens 210, a relay lens 220, and an imaging lens 230. The numerical apertures (NAs) of the coupling lens 210 and the imaging lens 230 may range from 0.45 to 0.55, and the NA of the relay lens 220 may range from 0.15 to 0.25. For example, in case of a GRIN lens with a lens diameter of 1 mm, the NAs of the coupling lens 210 and the imaging lens 230 may be 0.5 and the NA of the relay lens 220 may be 0.2.

The rotatable probe moving device 300 has a structure that not only enables the endoscope probe 200 to rotate but also supports fine adjustment of a horizontal movement and vertical movement of the endoscope probe 200. Thus, the rotatable probe moving device 300 controls planar movement (in X axis and Y axis directions) and the vertical movement in Z axis direction, which are important in the position relationship between the combined endoscope probe 200 and the objective lens of the microscope apparatus 100.

Referring to FIG. 3, the rotatable probe moving device 300 may include a probe holder holding an endoscope probe 200, a rotation mount 330 rotating finely the probe holder 310, and a translation stage 350 moving finely the rotation mount 330 in X axis, Y axis, and Z axis. The rotatable probe moving device 300 may be implemented with a combination of members controlling rotation and three-axis movement (vertical and horizontal movements), and detailed configurations thereof may be designed in various ways.

The wall glass capillary 400 is manufactured in consideration of an operation distance (400µm) of a light source that can be delivered to a living tissue through the GRIN lens. The wall glass capillary 400 may be, for example, a very thin glass tube with an inner diameter of 1.04 mm and an outer diameter of 1.2 mm. The wall glass capillary 400 may be implanted in an animal with being fixed to a plate designed to be suitable for holding a skull for fine axial adjustment. The lower end of the wall glass capillary 400 is closed, and the upper end is open so that the endoscope probe 200 can be inserted. The inner wall of the wall glass capillary 400 can be surface treated so that the endoscope probe 200 can move and rotate smoothly. The inside of the wall glass capillary 400 may be filled with a liquid having a specific refractive index in consideration of the refraction of a light from the endoscope probe 200.

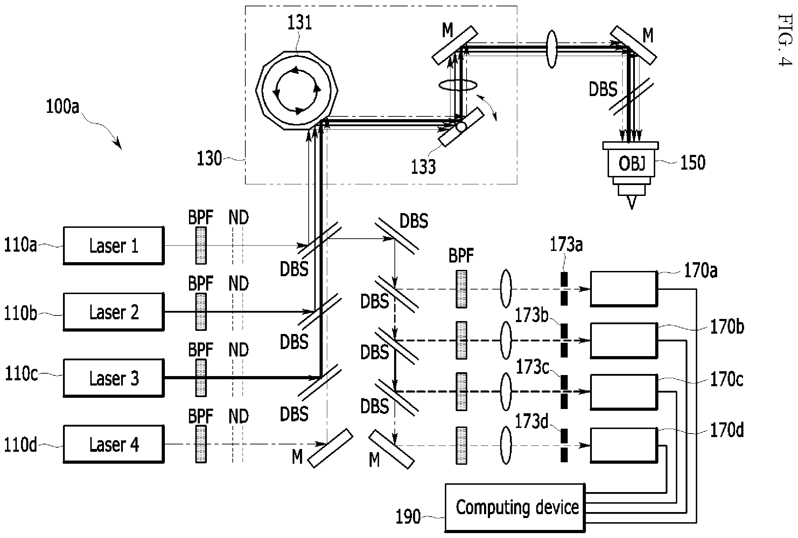

FIG. 4 is an exemplary confocal microscope device according to an embodiment.

Referring to FIG. 4, a confocal microscopy may be used in a microscope apparatus 100.

A confocal microscope device 100a includes laser light sources 110a, 110b, 110c, and 110d emitting lights of multiple wavelengths, a scanner 130 that scans lights from the laser light sources 110a, 110b, 110c, and 110d in the X-Y direction using a plurality of mirrors, an objective lens 150 that delivers the lights having passed through the scanner 130 to a target tissue labeled with a fluorescent material, photodetectors 170a, 170b, 170c, and 170d that detect fluorescence signals reflected from the focus of the objective lens 150 by wavelength using pinholes, and a computing device 190 that generates an image by combining the signals detected by the photodetectors 170a, 170b, 170c, and 170d.

Each of the laser light sources 110a, 110b, 110c, and 110d is composed of four laser light sources in the visible light band, and may be, for example, laser light sources with wavelengths of 405 nm, 488 nm, 561 nm, and 640 nm. The number and wavelengths of the laser light sources used as excitation light sources may be changed in various ways.

An optical path may be designed so that the light emitted from each of the laser light sources 110a, 110b, 110c, and 110d sequentially passes a beam pass filter (BPF), a gray filter (neutral density filter, NDF), and a beam splitter splitting a beam of light according to the wavelength and then is transmitted to the scanner 130. A mirror (M) may be used to change the optical path. The beam splitter may be a dichroic beam splitter (DBS).

The scanner 130 may include a polygonal rotation mirror 131 and a galvanometer mirror 133. The polygon rotation mirror 131 may scan the X axis and the galvanometer mirror 133 may scan the Y axis to generate an X-Y raster scan pattern. The scanner 130 makes a focal position of the objective lens to move quickly according to a laser scanning pattern.

The objective lens 150 delivers a light having passed through the scanner 130 into a target tissue labeled with a fluorescent material. A light emitted from a deep part of the tissue labeled with the fluorescent material (fluorescence signal) enters the objective lens 150 after passing through the endoscope probe 200 and then is transmitted to the scanner 130. The objective lens 150 may be implemented with an objective lens of 40x magnification, and may be set to have various fields of view by using lenses of proper magnifications.

The fluorescence signal is transmitted from the scanner 130 to the photodetectors 170a, 170b, 170c, and 170d. The photodetectors 170a, 170b, 170c, and 170d may be photomultiplier tubes (PMTs) separately provided for each wavelength, and the fluorescence signal of each wavelength is delivered to the photomultiplier tubes through pinholes 173a, 173b, 173c, and 173d formed on the confocal plane. The pinhole may be a fine hole or a slit formed on the confocal plane. The fluorescence signal transmitted from the scanner 130 may be split by wavelength through the dichroic beam splitters (DBSs). Each of the photodetectors 170a, 170b, 170c, and 170d converts an input fluorescence signal into an electrical signal and then transmits the electrical signal to the computing device 190.

The computing device 190 combines the electrical signals received from the photodetectors 170a, 170b, 170c, and 170d according to the laser scanning pattern and generates a microscopic image at the cellular level and molecular level. The computing device 190 may include a frame grabber. For example, the computing device 190 may acquire 2D images of the cellular level resolution, to which Z-axis partitioning can be applied, at a speed of 30 frames per second.

Particularly, when signals are transmitted from the photodetectors 170a, 170b, 170c, 170d in real time according to the movement on Z-axis or rotational movement of the endoscope probe 200, the computing device 190 can perform real-time imaging of fluorescent phenomenon occurring in the deep tissue of a living animal at the cellular level or molecular level.

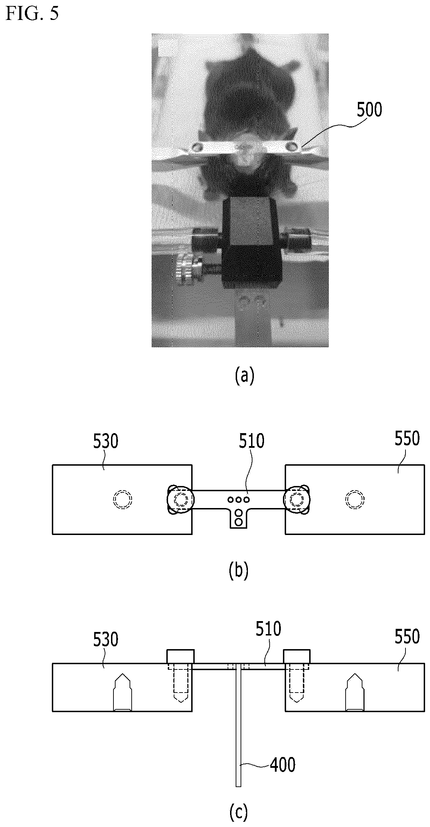

FIG. 5 is a diagram illustrating a skull attachment plate for implanting a wall glass capillary in a body according to an embodiment.

Referring to (a) of FIG. 5, the wall glass capillary 400 can be implanted in an animal with being attached to the plate 500. Alternatively, the implanted wall glass capillary 400 may be attached to the plate 500.

In order to perform fine axial adjustment with an endoscope probe 200 combined with the microscope apparatus 100, the position of a hole on the plate 500 into which at least one wall glass capillary is inserted is determined. In addition, the animal to which the wall glass capillary 400 is attached may be stereotactically fixed through fixing the plate 500 to the motorized stage. Due to the plate 500, the endoscope probe 200 and the wall glass capillary 400 are mechanically aligned, so that the endoscope probe 200 can be accurately, stably, and repeatedly inserted into the wall glass capillary 400.

Since a capillary phenomenon may occur due to bleeding caused by implanting a glass tube in a living animal, a wall glass capillary can be inserted into a living tissue with the bottom end being closed in the glass tube insertion technology. In particular, in the present invention, a microscopic image of living tissue can be acquired through a surgery method which inserts the wall glass capillary 400 attached to the plate 500 into the brain tissue, while physiological and behavioral characteristics of the living tissue are maintained.

The shape of the plate may be changed in various ways depending on the position where the wall glass capillary is inserted, the size and length of the wall glass capillary, the size and the part of an animal to which the wall glass capillary is inserted, and the structure of the stage to which the plate is fixed.

Referring to (b) of FIG. 5, the skull attachment plate 500 that fixes the wall glass capillary inserted into a brain tissue of a mouse may include, for example, a glass tube insertion plate 510, and stage fixing plates 530 and 550 connected to both ends of the glass tube insertion plate 510. The glass tube insertion plate 510 has at least one hole into which the wall glass capillary can be inserted.

Referring to (c) of FIG. 5, the wall glass capillary is attached and fixed through the hole of the glass tube insertion plate 510.

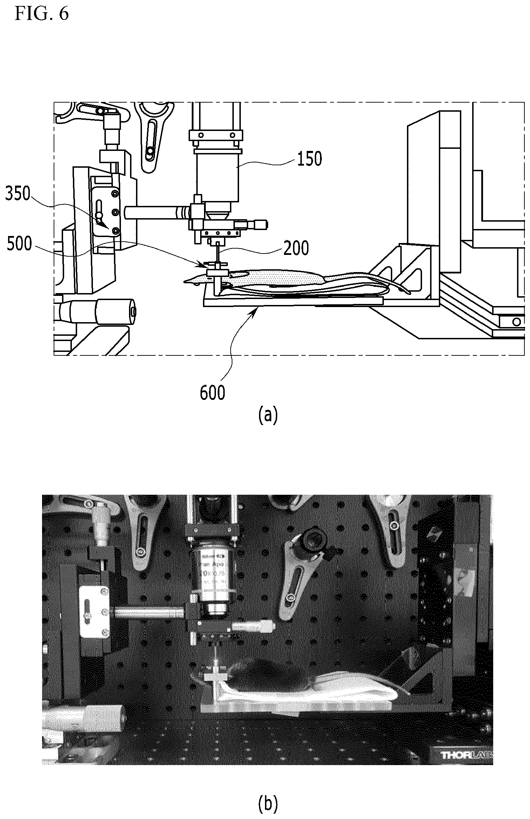

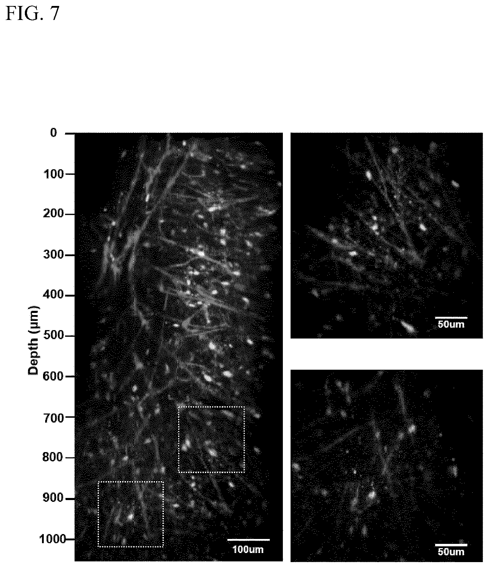

FIG. 6 is a diagram illustrating a case where a mouse is fixed to a stage of a microscopic image acquisition system according to an embodiment, and FIG. 7 is an exemplary image of a mouse brain tissue acquired by a microscopic image acquisition system according to an embodiment.

Referring to (a) of FIG. 6, a mouse to which a skull attachment plate 500 attached is placed on a stage 600 of a microscopic image acquisition system and the skull attachment plate 500 is stereotactically fixed on the stage 600. Thereafter, an endoscope probe 200 connected to a rotatable probe moving device 300 moves vertically and rotates, and a light having passed through an objective lens 150 is delivered to an endoscope probe 200 to scan the deep tissue around the wall glass capillary.

Actually, the mouse may be fixed on the stage of the microscopic image acquisition system as shown in (b) of FIG. 6.

For image acquisition, DyLight 594 conjugated Lectin antibody is injected into a CX3CR1-GFP mouse 1 hour before the experiment. Then, blood vessels are stained, and microglia cells in the brain tissue emit green fluorescent light. As a result, a fluorescence image as shown in FIG. 7 is acquired. A light with multiple wavelengths is emitted while simultaneously performing the vertical movement and rotation movement of the endoscope probe 200 and the vertical movement of the motor stage 600. As a result, a high resolution image at the cellular level can be acquired in an extended area as shown in FIG. 7.

Referring to FIG. 7, dual-color imaging of vessels stained with Lectin 549 antibody and GFP-expressing microglia can be performed through large-area and multichannel imaging of deep living tissue.

After an image is acquired, the coordinate and depth of the insertion into the brain tissue may be re-verified through tissue extraction and fixation.

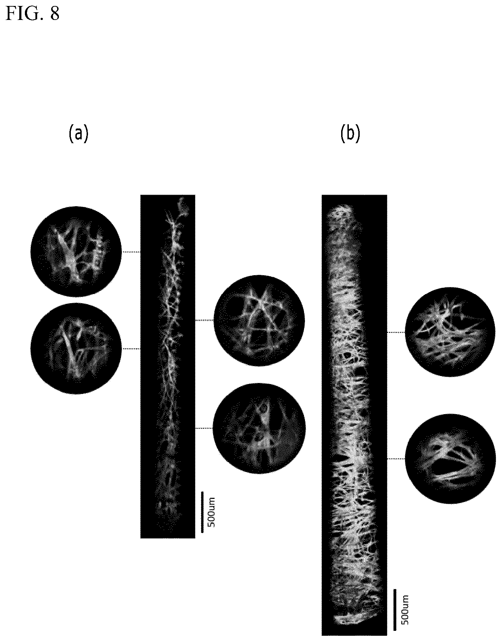

FIG. 8 is an exemplary large-area fluorescence image acquired according to an embodiment.

Referring to FIG. 8, a lens tissue sample coated with FITC fluorescent material is applied to a microscopic image acquisition system 10 that can stably and repeatedly insert an endoscope probe 200 along a thin glass tube, and, as a result, a large-area fluorescence image can be acquired through vertical movement and rotation of the endoscope probe 200.

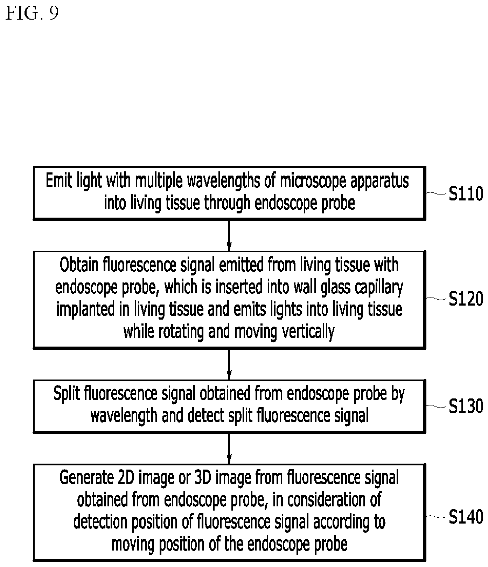

FIG. 9 is a flowchart of a microscopic image acquisition method according to an embodiment.

Referring to FIG. 9, a microscopic image acquisition system 10 emits a light with multiple wavelengths of a microscope apparatus 100 into a living tissue through an endoscope probe 200 (S110). The light having passed through an objective lens of the microscope apparatus 100 is transmitted to the endoscope probe 200.

The microscopic image acquisition system 10 obtains a fluorescence signal emitted from the living tissue with the endoscope probe 200, which is inserted into a wall glass capillary implanted in the living tissue and emits lights into the living tissue while rotating and moving vertically (S 120).

The microscopic image acquisition system 10 splits the fluorescence signal obtained from the endoscope probe 200 by wavelength and detects the split fluorescence signal (S130).

The microscopic image acquisition system 10 generates a 2D image or a 3D image from the fluorescence signal obtained from the endoscope probe 200, in consideration of a detection position of the fluorescence signal according to the moving position of the endoscope probe 200 (S140). The moving position of the endoscope probe 200 is determined by the rotational movement and vertical movement of the endoscope probe 200.

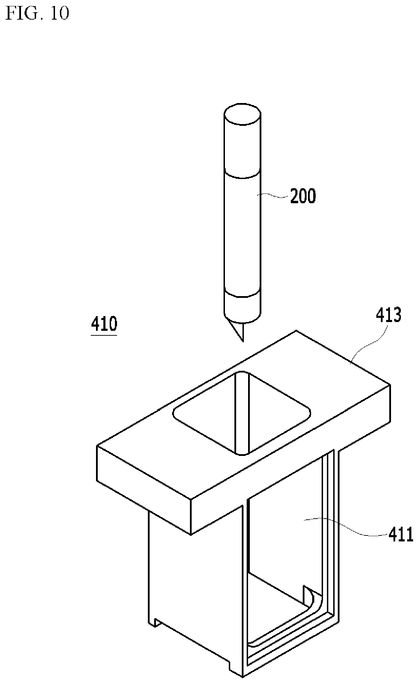

FIG. 10 is an exemplary implantable device implanted in a living body according to another embodiment.

Referring to FIG. 10, a thin glass tube 400 or a square-type window chamber 410 may be implanted in a tissue of a living animal.

The square-type window chamber 410 may be fabricated with a chamber 411 and an upper end wing 413. The chamber 411 has a rectangular tubular shape to form a certain space. The bottom of the chamber 411 can be made closed. Alternatively, the bottom of the chamber 411 can be made open, and the experimenter may attach a thin plate to the bottom surface of the chamber. A glass plate is attached to the side of the chamber 411. Alternatively, the side of the chamber 411 is made open, and the experimenter may attach a glass plate with an optic bond. A light from a rotatable endoscope probe 200 may be delivered to a tissue through the glass plate attached to the side of the chamber 411.

An upper end wing 413 includes a wing that fixes the chamber 411 and a passage through which the endoscope probe 200 is inserted into the chamber 411. For example, when the square-type window chamber 410 is implanted in a brain tissue, the upper end wing 413 can be stably attached to a head (skull). The upper end wing 413 may substitute for a plate with a complicated structure as shown in FIG. 3, or may be simply implemented.

For reference, in FIG. 10, as long as the endoscope probe 200 can be inserted into the square-type window chamber 410, the size of the endoscope probe 200 and that of square-type window chamber 410 may be variously changed.

The square-type window chamber 410 can image a wider plane than the wall glass capillary 400. Since the square-type window chamber 410 moves vertically while moving in parallel with the surface to which the glass plate is attached, the square-type window chamber 410 can image more extended deep tissue than the wall glass capillary 400.

As a result, the present invention enables repetitive access to deep tissues in a living animal by implanting a wall glass capillary 400 or square-type window chamber 410 in a body and inserting an endoscope probe into the implanted device. Particularly, using the wall glass capillary 400 or the square-type window chamber 410 allows multichannel imaging of more extended area in the deep tissue at the cellular level.

As described above, through fluorescence imaging of an animal tissue, the microscopic image acquisition system 10 can accurately observe, in real-time, phenomena occurring in the living tissue while maintaining physiological and behavioral characteristics.

Since it is possible to repeatedly acquire microscopic images of the same deep tissue in a living body, changes in the tissue of the living body over time can be observed repeatedly and in the long term.

Since the endoscope probe moves vertically and rotates, the microscopic image acquisition system 10 can acquire an extended image of a large area and can acquire not only 2D images but also 3D images.

The microscopic image acquisition system 10 can stably access the deep tissue that the conventional fluorescence endomicroscopy cannot access. Accordingly, the microscopic image acquisition system 10 can identify various diseases or biological phenomena occurring in various deep tissues including brain tissues.

The embodiments of the present invention described above are not implemented only through an apparatus and a method, but may be implemented through a program for realizing a function corresponding to the configuration of the embodiments of the present invention or a recording medium on which the program is recorded.

Although the embodiments of the present invention have been described in detail above, the scope of the present invention is not limited thereto, and various modifications and improvements by those skilled in the art using the basic concept of the present invention defined in the following claims are also provided. It belongs to the scope of rights.

## Claims

1. A microscopic image acquisition system comprising:
a microscope apparatus that scans light of multiple wavelengths, transmits the light to an objective lens, and detects a fluorescence signal reflected at a living tissue and transmitted to the objective lens;
an endoscope probe including lenses, which is inserted into an implantable device implanted in a living body, transmits an incident light from the objective lens to a side opening at a lower end, and transmits a fluorescence signal emitted from the living tissue labeled with a fluorescent material to the objective lens; and
a rotatable probe moving device that fixes the endoscope probe to rotate or vertically move the fixed endoscope probe.

2. The microscopic image acquisition system of claim 1, wherein the endoscope probe comprises a gradient index lens (GRIN lens) to transmit a light having passed through the objective lens, and a microprism to emit laterally a light transmitted from the GRIN lens.

3. The microscopic image acquisition system of claim 1, wherein the endoscope probe comprises a coupling lens, a relay lens, and an imaging lens.

4. The microscopic image acquisition system of claim 1, wherein the rotatable probe moving device comprises
a probe holder to fix the endoscope probe;
a rotation mount to interlock with the endoscope probe and to rotate the endoscope probe; and
a translation stage to vertically and horizontally move the rotation frame.

5. The microscopic image acquisition system of claim 4, wherein the rotatable probe moving device rotates the endoscope probe fixed to the probe holder by moving the rotation frame and vertically moves the endoscope probe by moving the translation stage.

6. The microscopic image acquisition system of claim 1, further comprising a stage implemented with a stereotactic system for an animal having implanted the implantable device,
wherein a plate to which the implantable device is attached is fixed to the stage.

7. The microscopic image acquisition system of claim 1, wherein the implantable device is implanted in the animal with being fixed to the plate.

8. The microscopic image acquisition system of claim 1, wherein the implantable device is filled with a liquid that has a specific refractive index to control refraction of a light emitted by the endoscope probe.

9. The microscopic image acquisition system of claim 1, wherein the microscope apparatus comprises:
laser light sources to emit the light of the multiple wavelengths;
a scanner to scan lights from the laser light sources using a plurality of mirrors;
the objective lens that transmits a light having passed through the scanner to the endoscope probe;
photodetectors that detect fluorescence signals having passed through pinholes formed on a confocal plane by wavelength, among the fluorescence signals transmitted from the endoscope probe; and
a computing device to generate an image by combining signals output from the photodetectors.

10. The microscopic image acquisition system of claim 1, wherein the microscope apparatus comprises a multi-photon microscopy.

11. The microscopic image acquisition system of claim 9, wherein the computing device generates a fluorescence image, expressing the fluorescent material labeled on a living tissue of a specific depth and excited by the laser light sources while the endoscope probe rotates.

12. The microscopic image acquisition system of claim 9, wherein the computing device stereoscopically generates a fluorescence image of the living tissue based on a rotational and a vertical movement position of the endoscope probe.

13. The microscopic image acquisition system of claim 1, wherein the implantable device is a wall glass capillary or a square-type window chamber that are implemented with a passage into which the endoscope probe is inserted.

14. A method for acquiring a microscopic image by a microscopic image acquisition system, the method comprising:
emitting a light of multiple wavelengths generated by a microscope apparatus into a living tissue through an endoscope probe; and
generating a fluorescence image using a fluorescence signal emitted from the living tissue, wherein the fluorescence image expresses a fluorescent material labeled on the living tissue and excited by a multi-wavelength laser,
wherein the light of multiple wavelengths is transmitted from an objective lens of the microscope apparatus to a lens of the endoscope probe and emitted to a living tissue around a side opening of the endoscope probe,
wherein the fluorescence signal is transmitted from the lens of the endoscope probe to the objective lens, and
wherein the endoscope probe is inserted into a wall glass capillary or a square-type window chamber implanted in the living tissue, and rotates and moves vertically.

15. The method of claim 14, wherein generating the fluorescence image comprises stereoscopically generating a fluorescence image of the living tissue based on rotational and vertical movement positions of the endoscope probe.

16. The method of claim 14, wherein the microscope apparatus comprises a confocal microscopy or a multi-photon microscopy.
